# EUROPEAN PATENT SPECIFICATION

(11) **EP 1 588 679 B1**
(45) Date of publication and mention of the grant of the patent: **12.09.2007**
(21) Application number: 04101600.7
(22) Date of filing: 19.04.2004
(51) Int. Cl.: A61F 5/44, A61J 1/00

(54) **Sterilizable bag for irrigation solutions**
Sterilisierbarer Beutel für Irrigationslösung
Sac stérilisable pour solution d'irrigation

(43) Date of publication of application: 26.10.2005
(73) Proprietor: B. BRAUN MELSUNGEN AG, 34212 Melsungen (DE)
(72) Inventor: Rew, Margaret, East Sheen, London SW 14 8 AN (GB); Loretti, Maurice, 1219 Chatelaine (CH); Djokic, Savka, 1007 Lausanne (CH)
(74) Representative: Jönsson, Hans-Peter

(56) References cited:
- EP-A- 0 092 528
- WO-A-83/02061
- DE-A- 2 950 791
- US-A- 5 195 980

## Description

The invention relates to a sterilizable bag for medical solutions, particularly irrigation solutions, its use for storage and application of irrigation solutions and recollecting liquid samples for analysis, and a process of bladder lavage or irrigation.

Catheters have been used for centuries. The catheter designed by Dr. Frederick Foley in 1930 is still the commonly used catheter design today. Although the use of catheters especially with elderly patients is well-established, the use of long-term catheters as a means of managing incontinence raises serious problems.

Catheter-associated urinary tract infections account for a large number of hospital-acquired infections. Forty-four per cent of hospitalized patients with catheters have been found to develop significant bacteruria within 72 hours of catheterization (Crow et al. A Study of Patients with an In-dwelling Urinary Catheter and Related Nursing Practice, Nursing Practice Research Unit, Guildfford 1986). Some of these infections result in complications including pyelonephritis, epididymitis, abscess formation and chronic renal failure (Warren et al., J. Infectious diseases 155, 1987, 11518). This leads to increased morbidity and mortality. Catheter blockage, which may lead to such conditions as mentioned above, represents one of the most common complications associated with infections (Elves and Feneley, Br. J. Urol. 80, 1997, 15).

There is a clear correlation between the number of times the drainage system is disconnected and the rate of infection (Getliffe and Dolman, Promoting Continence: A Clinical and Research Resource. Baillière-Tindall, London 1997). It is important, therefore, to keep the drainage system closed as much as possible.

Conventional bags used for catheter irrigations, e.g. the URO-TAINER^{®} system available from B. Braun Medical AG, CH, is made of a single-chamber bag with a closable outlet tube which can be connected to an indwelling catheter.

However, effective rinsing and sterilizing of indwelling catheters and/or the bladder interior often requires repeated irrigations with the same or different medical solutions. This means that the sensitive connection between the indwelling catheter and the connector tube of the bag has to be broken for each change of bag connection. This poses problems for the sterility of the connection with potentially disastrous implications on the patient's health due to infection with yeasts, bacteria, mycelia, and fungi.

Disadvantages and risks of conventional irrigation bags and irrigation treatment have been outlined in a survey (M. Rew, Infect Immun, 25, 1999, 552) which also reports specific indications for the use and non-use of irrigation solutions. These disadvantages include urinary tract infections and encrustation of the catheter leading to blockage of the catheter lumen, bypassing or retention of urine as well as pain.

WO 83/02061 A1 discloses a multiple chamber system for peritioneal dialysis with an empty bag chamber and a chamber filled with dialysis solution.

It is an object of the present invention to provide a sterilizable bag for medical solutions, especially suitable for bladder lavage and irrigation of indwelling catheters which avoids the above mentioned problems. In particular the increased risk of infection due to repeated disconnections of the catheter system should be minimized.

It is a further object of the invention to provide a convenient means of separately collecting samples of liquids from the urinary tract for urological, biological and/or chemical analysis without an increased risk of infection.

The object is primarily solved by a sterilizable bag (1) comprising at least two chambers (2,3), said chambers having outlets leading into closable inner tubes (5) which are connected to each other by a connector (6) to a common outer tube (7), said common outer tube having a means (8) for connecting to an indwelling catheter, characterized in that at least two chambers are fully or partly filled with medical solutions and each chamber has a volume of 10 to 60 ml.

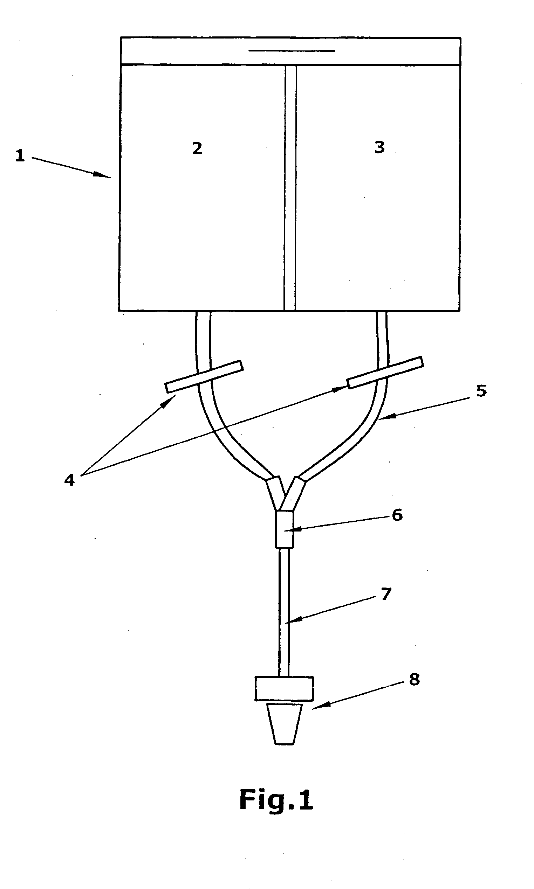

Fig. 1 shows a schematic view of a bag according to the invention.

This system of irrigation by catheter poses no technical problems in particular and its availability is immediate. In particular, the bag according to the invention allows for subsequently applying medical solutions, especially irrigation solutions, identical or different, without the need to open the catheter system. The risk of infection due to repeated disconnections which is prominent in the prior art is thus minimized significantly, at the same time maximizing the rinsing/lavaging effect of the application.

The irrigation may be effected anywhere, giving maximum flexibility to the treatment. In addition, the simplicity of the system does not require a qualified person to carry out this kind of irrigation which can be done by the patient himself or a member of his family.

It is a system especially suitable for those institutions in which there are few qualified personnel and for those patients who carry the catheter at home.

The bag according to the invention may advantageously be designed in that said Y-connector is a valve-switch either opening an interconnection between all or at least two inner tubes or between one inner tube and the outer tube or closing all interconnections. In this embodiment no e.g. clamps are necessary for the separate closing or opening of each inner tube. The switch may be operated by turning a protrusion on the front side of the Y-connector or any other means.

In another embodiment, as illustrated by Fig. 1, said inner tubes are closable by clamps 4.

The bag of the present invention may in principle be produced from any sterilizable and liquid-tight material. Preferred and advantageous are flexible and/or transparent plastic materials such as PVC or a multilayer film structure.

Conventional bags for bladder lavage and irrigation, such as the URO-TAINER^{®} system have an inner volume for the reception of medical solutions of 50 or 100 ml, respectively. A catheter lumen, however holds little more than 4 ml and therefore most of the volume in the first line causes discomfort by exceeding pressure within the bladder. The bag of the present invention therefore comprises chambers that have a volume of 10 to 60, preferably 20 to 40, in particular 25 to 35 ml, to avoid these shortcomings of the prior art.

In one embodiment of the invention each of the chambers of the bag contains a different medical solution. This opens the opportunity to exploit synergistic effects which occur by e.g. combining treatment with Suby G^{®}-solution and chlorhexidine solution with respect to removal of crystalline impurities and sterility in terms of bacteria count. Crystalline impurities present and/or developed in indwelling catheters may comprise urates, uric acid, phosphates, triple phosphates, oxalates and amorphous salts and tend to block the catheter if they are not removed on a regular basis.

In another preferred embodiment of the invention each of the chambers contains an identical medical solution. This provides for e.g. a better rinsing of the catheter and the bladder.

The chambers of the bag of the present invention are to be fully or partly filled, preferably including the case that at least two, but not all of the chambers may contain a medical solution. In this latter embodiment the empty chamber may serve as recollection chamber of a further sample for analysis, or it may be equipped with a filling device to be filled in-situ by the hospital personnel.

The bag of the present invention may in principle advantageously contain any medical. Particularly preferred fillings, however, are irrigation solutions.

The irrigation solutions may e.g. be chosen from commonly known solutions such as mandelic acid, citric acid, chlorhexidine, sodium chloride, and mixtures thereof. Aqueous mandelic acid 1 %, citric acid 3.23 % (Suby G^{®}), citric acid 6 % (Solution R^{®}), chlorhexidine 1:5000 and sodium chloride 0.9 % solution (saline) are among the most preferred to be used in the chambers of the bag.

The bag of the invention is mainly used for storage and application of irrigation solutions for rinsing and/or disinfecting indwelling catheters. It may as well in combination or alternatively be used for flushing indwelling catheters and the bladder and recollecting essentially liquid samples for urological, biological and/or chemical analysis with a minimized risk of infection due to exposure of the catheter system to the non-sterile environment

In order to achieve the competing objects of the invention, i.e. effective combined and/or synergistic bladder lavage and irrigation on the one hand and minimized risk of infection on the other, the present invention contrary to conventional bags allows for a process of bladder lavage or irrigation comprising
a) disconnecting and removing an e.g. urine bag from an indwelling catheter,
b) liquid-tight connecting the outer tube of a bag as outlined above with the indwelling catheter
c) introducing said medical solution contained in a first chamber of said bag and closing the respective inner tube, or, optionally closing the interconnection of said inner tube or all interconnections by switching said Y-connector valve switch,
d) maintaining said medical solution in the catheter and optionally, the bladder
e) recovering said used medical solution, which eventually contains solids, in said first chamber of the bag,
f) repeating steps c) to e) with medical solution contained in a second chamber of said bag
g) optionally repeating steps c) to e) with solutions contained in further chambers of said bag
h) disconnecting and replacing said bag.

These steps may then be followed by a step of urological, biological and/or chemical analysis of the recovered liquids and/or solids.

### Example 1

A bag was designed and produced according to Fig. 1, made of PVC and having two separate chambers, separated by a welding of 5 mm width. Each chamber had a width of 40 mm, a length of 118 mm and an inner volume of 2x60 ml. The sterile chambers were filled with 2x30 ml sterile Suby G^{®}-solution and were connected to an outer tube via separate flexible inner tubes and a common Y-connector. The 60 mm long inner tubes were closed by openable clamps. The 60 mm long outer tube was at its free end equipped with a standard means for connection to an indwelling catheter.

### Example 2

Bags of Example 1 were sterilized and evaluated in a clinical test with randomly chosen patients requiring a urinary tract catheter. The patients were cared for by a total of 36 nurses who have been asked for their evaluation of the following properties:
a) Handling of the material of the product:
   35 % Excellent; 55 % Satisfactory; 10 % Unsatisfactory
b) Usefulness of the hole for hanging up the bag:
   64 % Yes; 36 % No
c) Size of the chambers to manipulate:
   69 % Excellent; 28 % Satisfactory; 3 % Unsatisfactory
d) Ease of opening the closure ring:
   42 % Excellent; 46 % Satisfactory; 12 % Unsatisfactory
e) Ease of manipulating the clamps:
   31 % Excellent; 50 % Satisfactory; 19 % Unsatisfactory
f) Ease of performing the instillation procedure:
   37 % Excellent; 54 % Satisfactory; 9 % Unsatisfactory
g) Overall rating:
   56 % Excellent; 41 % Satisfactory; 3 % Unsatisfactory
h) Bag of the invention compared to single chamber "Uro-Tainer"^{®} used in the past:
   71 % Better; 26 % About the same; 3 % Not as good
i) Satisfaction with the prospect of using the bag of the invention in the future:
   97 % Yes; 3 % No
j) Advantage or disadvantage of the smaller amount of fluid in the chambers of the invention compared to the prior art:
   13 nurses stated that it was an advantage; no one stated that it was a disadvantage.

## Claims

1. Sterilizable bag (1) comprising at least two chambers (2,3), said chambers having outlets leading into closable inner tubes (5) which are connected to each other by a connector (6) to a common outer tube (7), said common outer tube having a means (8) for connecting to an indwelling catheter, **characterized in that** at least two chambers are fully or partly filled with medical solutions and each chamber has a volume of 10 to 60 ml.

2. Bag (1) according to claim 1, **characterized in that** said connector (6) is a valve-switch either opening an interconnection between all or at least two inner tubes (5) or between one inner tube and the outer tube or closing all interconnections.

3. Bag (1) according to claim 1 or 2 wherein said inner tubes (5) is closable by clamps (4).

4. Bag (1) according to any one of claims 1 to 3, comprising a flexible and/or transparent plastic material, in particular PVC or a multilayer film structure.

5. Bag (1) according to any one of claims 1 to 4 **characterized in that** said chambers (2,3) have a volume 20 to 40, in particular 25 to 35 ml.

6. Bag (1) according to any one of claims 1 to 5, **characterized in that** said chambers (2,3) contain different medical solutions.

7. Bag (1) according to any one of claims 1 to 5, **characterized in that** said chambers (2,3) contain identical medical solutions.

8. Bag (1) according to any one of claims 1 to 5, **characterized in that** at least two, but not all of the chambers (2,3) contain a medical solution.

9. Bag (1) according to any one of claims 6 to 8, **characterized in that** the medical solutions are irrigation solutions.

10. Bag (1) according to claim 9, **characterized in that** the irrigation solutions are chosen from solutions of mandelic acid, citric acid, chlorhexidine, sodium chloride, and mixtures thereof.

11. Bag (1) according to claim 1, **characterized in that** said connector (6) is a Y-connector in case of the presence of two chambers (2, 3).

## Patentansprüche

1. Sterilisierbarer Beutel (1), der wenigstens zwei Kammern (2, 3) umfasst, wobei die Kammern Ausgänge haben, die in verschließbare Innenrohre (5) führen, welche durch ein Verbindungsteil (6) miteinander und mit einem gemeinsamen Außenrohr (7) verbunden sind, wobei das gemeinsame Außenrohr ein Mittel (8) zum Anschließen eines Dauerkatheters aufweist, **dadurch gekennzeichnet, dass** wenigstens zwei Kammern vollständig oder teilweise mit medizinischen Lösungen gefüllt sind und die Kammer ein Volumen von 10 bis 60 ml hat.

2. Beutel (1) gemäß Anspruch 1, **dadurch gekennzeichnet, dass** das Verbindungsteil (6) ein Ventilschalter ist, der entweder eine Verbindung zwischen allen oder wenigstens zwei Innenrohren (5) oder zwischen einem Innenrohr und dem Außenrohr öffnet oder alle Verbindungen schließt.

3. Beutel (1) gemäß Anspruch 1 oder 2, wobei die Innenrohre (5) durch Quetschhähne (4) verschließbar sind.

4. Beutel (1) gemäß einem der Ansprüche 1 bis 3, der ein flexibles und/oder transparentes Kunststoffmaterial, insbesondere PVC oder eine mehrschichtige Filmstruktur, umfasst.

5. Beutel (1) gemäß einem der Ansprüche 1 bis 4, **dadurch gekennzeichnet, dass** die Kammern (2, 3) ein Volumen von 20 bis 40, insbesondere 25 bis 35 ml, haben.

6. Beutel (1) gemäß einem der Ansprüche 1 bis 5, **dadurch gekennzeichnet, dass** die Kammern (2, 3) verschiedene medizinische Lösungen enthalten.

7. Beutel (1) gemäß einem der Ansprüche 1 bis 5, **dadurch gekennzeichnet, dass** die Kammern (2, 3) identische medizinische Lösungen enthalten.

8. Beutel (1) gemäß einem der Ansprüche 1 bis 5, **dadurch gekennzeichnet, dass** wenigstens zwei, aber nicht alle der Kammern (2, 3) eine medizinische Lösung enthalten.

9. Beutel (1) gemäß einem der Ansprüche 6 bis 8, **dadurch gekennzeichnet, dass** die medizinischen Lösungen Spüllösungen sind.

10. Beutel (1) gemäß Anspruch 9, **dadurch gekennzeichnet, dass** die Spüllösungen aus Lösungen von Mandelsäure, Zitronensäure, Chlorhexidin, Natriumchlorid und Gemischen davon ausgewählt sind.

11. Beutel (1) gemäß Anspruch 1, **dadurch gekennzeichnet, dass** das Verbindungsteil (6) im Falle der Anwesenheit von zwei Kammern (2, 3) ein Y-Verbindungsteil ist.

## Revendications

1. Sac stérilisable (1) comprenant au moins deux chambres (2, 3), lesdites chambres comportant des orifices de sortie menant à des tubes intérieurs fermables (5) qui sont reliés entre eux par un raccord (6) à un tube extérieur commun (7), ledit tube extérieur commun comportant un moyen (8) permettant de le relier à un cathéter à demeure, **caractérisé en ce qu'**au moins deux chambres sont entièrement ou partiellement remplies de solutions médicales et chaque chambre a un volume de 10 à 60 ml.

2. Sac (1) selon la revendication 1, **caractérisé en ce que** ledit raccord (6) est un commutateur qui ouvre soit une interconnexion entre tous ou au moins deux tubes intérieurs (5) soit entre un tube intérieur et le tube extérieur, ou qui ferme toutes les interconnexions.

3. Sac (1) selon la revendication 1 ou 2, dans lequel ledit tube intérieur (5) peut être refermé par des pinces (4).

4. Sac (1) selon l'une quelconque des revendications 1 à 3, comprenant une matière plastique flexible et/ou transparente, en particulier du PVC ou une structure de film multicouche.

5. Sac (1) selon l'une quelconque des revendications 1 à 4, **caractérisé en ce que** lesdites chambres (2, 3) ont un volume de 20 à 40, en particulier de 25 à 35 ml.

6. Sac (1) selon l'une quelconque des revendications 1 à 5, **caractérisé en ce que** lesdites chambres (2, 3) contiennent des solutions médicales différentes.

7. Sac (1) selon l'une quelconque des revendications 1 à 5, **caractérisé en ce que** lesdites chambres (2, 3) contiennent des solutions médicales identiques.

8. Sac (1) selon l'une quelconque des revendications 1 à 5, **caractérisé en ce que** au moins deux, mais pas toutes les chambres (2, 3) contiennent une solution médicale.

9. Sac (1) selon l'une quelconque des revendications 6 à 8, **caractérisé en ce que** les solutions médicales sont des solutions d'irrigation.

10. Sac (1) selon la revendication 9, **caractérisé en ce que** les solutions d'irrigation sont choisies parmi les solutions d'acide mandélique, d'acide citrique, de chlorhexidine, de chlorure de sodium et leurs mélanges.

11. Sac (1) selon la revendication 1, **caractérisé en ce que** ledit raccord (6) est un raccord en Y dans le cas de la présence de deux chambres (2, 3).
